Europäisches Patentamt

**European Patent Office**

Office européen des brevets

(11) Publication number : **0 323 748 B1**

(12) **EUROPEAN PATENT SPECIFICATION**

(45) Date of publication of patent specification :
**04.03.92 Bulletin 92/10**

(21) Application number : **88312332.5**

(22) Date of filing : **28.12.88**

(51) Int. Cl.$^5$ : **C10G 32/02**

(54) **Bioelectrochemical desulphurisation of petroleum.**

(30) Priority : **31.12.87 KR 1557387**

(43) Date of publication of application :
**12.07.89 Bulletin 89/28**

(45) Publication of the grant of the patent :
**04.03.92 Bulletin 92/10**

(84) Designated Contracting States :
**DE FR GB IT NL SE**

(56) References cited :
**DD-A- 138 780**
**US-A- 2 641 564**

(73) Proprietor : **KOREA ADVANCED INSTITUTE**
**OF SCIENCE AND TECHNOLOGY**
**39-1 Haweolgog-dong Sungbuk-gu**
**Seoul (KR)**

(72) Inventor : **Kim, Byung-Hong**
**264-440 Yimun 2-dong**
**Dongdaemun-gu Seoul (KR)**
Inventor : **Kim, Tae-Sung**
**39-1 Haweolgog-dong**
**Sungbuk-gu Seoul (KR)**
Inventor : **Kim, Hae-Yeong**
**39-1 Haweolgog-dong**
**Sungbuk-gu Seoul (KR)**

(74) Representative : **Perry, Robert Edward et al**
**GILL JENNINGS & EVERY 53-64 Chancery**
**Lane**
**London WC2A 1HN (GB)**

EP 0 323 748 B1

## Description

Field of the Invention

This invention relates to the microbial desulphurisation of petroleum.

Background of the Invention

The use of petroleum is expected to increase, despite the diminishing world reserves of high quality crude oils. The sulphur oxides generated from the combustion of petroleum products may play a major role in the formation of "acid rain" which produces lasting detrimental effects on aquatic as well as terrestrial ecosystems. The demand for low sulphur petroleum has been intensified by increasingly stringent regulatory standards for reduced levels of sulphur oxide in atmospheric emissions. For compliance with the regulatory standard, fuels having a high sulphur content must be subjected to either pre- or post-combustion desulphurisation.

Processes for the desulphurisation of oil which are currently in use, both chemical and physical, are inadequate to solve the problem. Desulphurisation of heavy oil by hydrogenation or by other chemical procedures requires special catalysts and/or extremely high temperature or pressure, making the process very expensive. Heavy metals in petroleum limit the utility of catalytic hydrodesulphurisation processes, as they poison the catalyst. Since both sulphur compounds and heavy metals are concentrated during refinery, sulphur removal from the residual fuel oil is even more difficult. These disadvantages of conventional desulphurisation processes, and the increasing concern about acid rain have stimulated interest in microbial sulphur transformations and microbial precombustion desulphurisation.

Aromatic-thiophene derivatives are virtually always found in crude oil. Dibenzothiophene (DBT) has therefore been used as a model compound in the investigation of crude oil desulphurisation. DBT-utilising aerobic bacteria such as Pseudomonas sp., Beijerinkia sp. and Bacillus sulfasportare have been used in attempts to remove organic sulphur compounds from petroleum and its products in water-soluble form (Malik, K.A. (1978) Process Biochem., 13(9), 10); this process is not economically feasible, and the air supply necessary for the oxidation of sulphur compounds by aerobic bacteria constitutes a hazard.

The possibility of using anaerobic bacteria for the removal of sulphur compounds has been reported. Davis and Updevatt, Bacteriol. Rev. 18 (1954) 215, attempted the microbial desulphurisation of crude oil by certain sulphate-reducing bacteria, but not successfully.

US-A-2641564 discloses the use of sulphate-reducing bacteria with high hydrogenase activities, to reduce organic sulphur in petroleum to hydrogen sulphide. The reactions were believed to be generally as follows:

$$R\text{-}S\text{-}R' + H_2 \to RSH + R'H \text{ (or } RH + R'SH)$$
$$R\text{-}S\text{-}R' + 2H_2 \to RH + H_2S + R'H$$

Kurita et al, J. Gen. Appl. Microbiol. 17 (1971) 185, disclose the isolation of bacterial cultures which produce $H_2S$ from organic sulphur compounds in petroleum, under anaerobic conditions. The isolates were also found to produce $H_2S$ from hydrogenated residue oil, crude oil and asphaltene. Methyl viologen as an electron mediator was essential for the reaction.

Kohler et al, Zbl. Mikrobiol., 139 (1984) 239, report that a selected Desulfovibrio strain utilising molecular hydrogen can reduce nine sulphur compounds found in petroleum to $H_2S$, and that the degree of desulphurisation is related to growth conditions and hydrogenase activity of the cultures. Eckart et al, Zbl. Mikrobiol. 141 (1986) 291, used the mixed cultures from different sediments for anaerobic desulphurisation of Romashkino petroleum. The main components of these mixed cultures were Desulfovibrio sp; concomitant strains were found to be Micrococci, Bacilli and Clostridia. The microbial desulphurisation processes utilising anaerobic bacteria described above have a particular drawback: hydrogen gas has to be supplied to the system for the reductive desulphurisation of organic sulphur compounds in petroleum by sulphate-reducing bacteria. DD-C-0138780 describes a method for the microbiological desulfurisation of mineral oil, which is conducted at an $rH_2$ value of 1.0 to 7.0 and a redox potential of -200 to -450 mV.

Summary of the Invention

The invention comprises the utilisation of electrochemical energy as a source of reducing equivalent instead of hydrogen gas in the microbial desulphurisation of fossil fuels such as petroleum, and coal-utilising sulphate-reducing bacteria as a catalyst.

The novel method is conducted in the cathode compartment of a partitioned cell.

Description of the Invention

Micro-organisms have been isolated from soil samples using Postgate medium E, and cultivated in Postgate medium C. The compositions of Postgate media C and E are as follows:

```
Medium C (per litre)
-------------------------------------------
KH₂PO₄                                   0.5 g
NH₄Cl                                    1.0 g
Na₂SO₄                                   4.5 g
CaCl₂.6H₂O                               0.06 g
MgSO₄.7H₂O                               0.06 g
Sodium lactate                          6.0 g
Yeast extract                           1.0 g
FeSO₄.7H₂O                              0.004 g
Sodium citrate.2H₂O                     0.3 g
pH                                      7.5
```

```
Medium E (per litre)
-------------------------------------------
KH₂PO₄                                   0.5 g
NH₄Cl                                    1.0 g
Na₂SO₄                                   1.0 g
CaCl₂.6H₂O                               1.0 g
MgSO₄.7H₂O                               2.0 g
Sodium lactate                          3.5 g
Yeast extract                           1.0 g
Ascorbic acid                           0.1 g
Thioglycolic acid                       0.1 g
FeSO₄.7H₂O                               0.5 g
pH                                      7.6
```

Culture ware made in pressure tubes and serum vials with a 5% inoculum at 30°C for mesophilic sulphate-reducing bacteria. Reference cultures were obtained from the National Collection of Industrial and Marine Bacteria (NCIMB). These were Desulfovibrio vulgaris NCIMB 8303 and Desulfovibrio desulfuricans NCIMB 8310. Strictly anaerobic procedures were maintened in all experiments, according to the method of Kim et al, Appli. Environ. Microbiol., 48 (1984) 764.

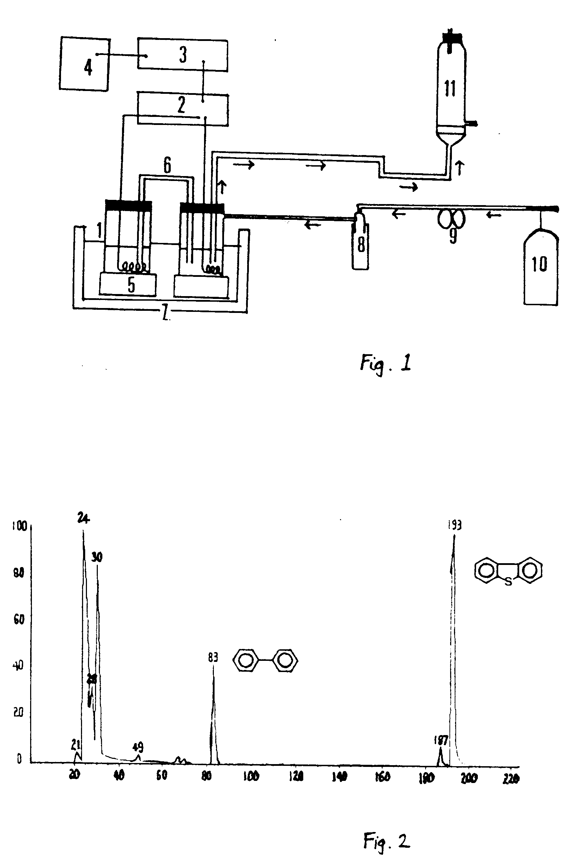

A two-compartment type cell similar to that used by Kim et al, Biotechnol. Lett. 10(2) (1988) 123, was used for the controlled supply of electrochemical energy. The electrochemical cell system is shown diagrammatically in the accompanying Figure 1 which shows an electrochemical cell 1, a potentiostat 2, an ammeter 3, a recorder 4, an immersed magnetic stirrer 5, an agar bridge 6, a vibrating water bath 7, a gas purifying oven 8, a low pressure regulator 9, a nitrogen gas supply 10, and a hydrogen sulphide absorber 11.

The two-half cells were held together by a U-shaped agar bridge. Platinum wire or carbon material was used as the electrode in the electrochemical system. The cathode compartment comprised a reaction mixture of sulphate-free medium with 2 mM methyl viologen, cell suspensions of sulphate-reducing bacteria, and petroleum (or 0.1% DBT). Methyl viologen was used for the efficient electron transfer between electrodes and bacterial cells, as an electron mediator. The reaction mixture was stirred and purged with oxygen-free nitrogen.

Working and counter-electrodes were immersed in the aqueous phase, before an electric potential was applied via the potentiostat connected to an ammeter. The current was recorded continuously. The sulphate-free medium comprised (per litre):

| | |
|---|---|
| $KH_2PO_4$ | 0.5 g |
| $NH_4Cl$ | 1.0 g |
| $CaCl_2.6H_2O$ | 0.06 g |
| Sodium lactate | 6.0 g |
| Yeast extract | 1.0 g |
| 0.2% resazurin | 1.0 g |
| pH | 7.4 |

The sulphur contents of crude oil and its products were determined by the bomb method (ASTM Standard 1976, General bomb method, D 129-64). Hydrogen sulphide evolved during the reaction of microbial desulphurisation was absorbed by a starch solution and titrated iodometrically by the Tutwiler method; see J. Am. Chem. Soc. 23 (1901) 173.

The following Examples further illustrate the present invention.

Example 1

DBT was used as a model compound for the selection of bacterial strains to be used in petroleum desulphurisation. Cultures grown for 2 days were anaerobically harvested and suspended in the given (anaerobic) sulphate-free medium. The cell suspensions were placed in a serum vial. 2 mM methyl viologen and 0.1% (w/v) DBT were added, followed by incubation at 30°C for 6 days on a rotary shaker. The headspace of the vial was filled with hydrogen gas. After the reaction, the reaction mixture was extracted by n-butanol for analyses by gas chromatographic and spectroscopic methods.

As shown in Table 1, DBT recovered after the treatment for 6 days ranged from 58% to 94%, whilst 96% of DBT was recovered from the control tubes. These results show that the reduction of DBT is not catalysed by methyl viologen and hydrogen. Among the cultures tested, Desulfovibrio sp. M6 isolated from soil showed the highest ability to degrade DBT. Desulfovibrio sp. M6 was used in further experiments to identify the degradation products of DBT by GC-mass spectroscopic and infrared spectroscopic methods.

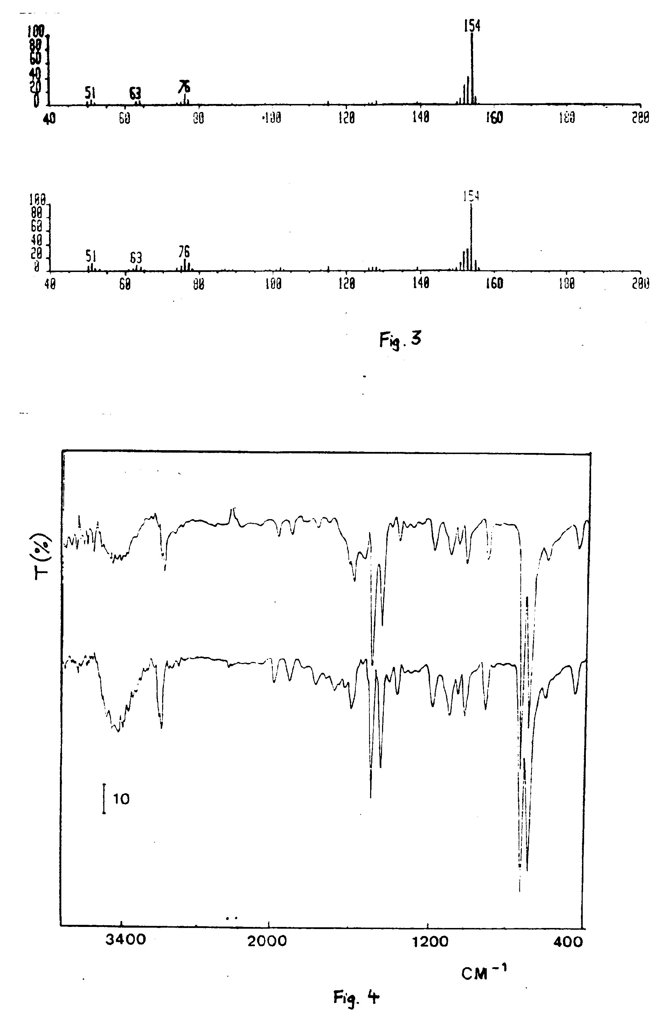

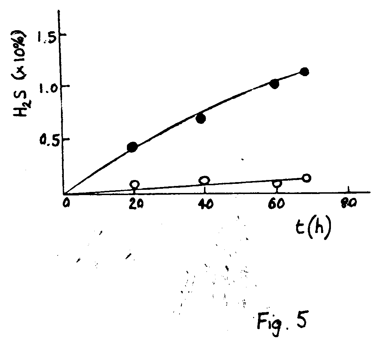

Figure 2 shows the gas chromatogram of dibenzothiophene treated in the system and extracted by n-butanol. Two peaks, at retention times of 83 and 187 seconds, appear in addition to the DBT peak at 193 seconds. The new peak at 83 seconds was analysed; mass spectrometry (Fig. 3, second plot) showed that the DBT degradation compound separated at 83 seconds is biphenyl (Fig. 3 first plot is the standard). This was substantiated, after separation on a preparative thin layer chromatogram, by infrared spectroscopic data of T (transmission) against wavelength (Fig. 4, lower plot), by comparison with a biphenyl standard (Fig. 4, upper plot).

Figure 5 is a plot showing the kinetics of $H_2S$ evolution by Desulfovibrio sp. M6 from crude oil against time (t). The heavy circles represent the cathode department.

## Table 1

### Degradation of DBT

| Reaction Time (days) | Control 1 | Control 2 | Relative DBT concentration (%) | | | | | | |
|---|---|---|---|---|---|---|---|---|---|
| | | | NCIMB | | Isolates | | | | |
| | | | 8303 | 8310 | M6 | M8 | S1 | SA | 5 |
| 0 | 100 | 100 | 100 | 100 | 100 | 100 | 100 | 100 | 100 |
| 4 | 97 | 98 | 95 | 92 | 87 | 98 | 90 | 90 | 90 |
| 6 | 96 | 96 | 85 | 81 | 58 | 94 | 83 | 79 | 88 |

Control 1: DBT only,

Control 2: DBT + 2 mM methyl viologen

Example 2

Desulfovibrio sp. M6 was used, to test its ability to reduce organic sulphur found in petroleum, with electrochemically-supplied reducing power. 35 ml Kuwait crude oil containing about 3% sulphur were placed in the cathode compartment of the illustrated cell, and the same volume of Desulfovibrio sp. M6 cell suspension in sulphate-free medium C with 2 mM methyl viologen was added. A platinum electrode having a surface area of $0.5 \text{ cm}^3$ was used. The cathode compartment was connected to the anode through a 10% KCl agar bridge, and a potential of 2.5 volt was applied using a potentiostat for 6 days at 30°C. The sulphur content was then analysed by the bomb method. The $H_2S$ gas evolved during the reaction was also measured by the Tutwiler method. The electrochemical cell containing the reaction solution was vigorously mixed, while the electrode was completely immersed in the aqueous phase.

Table 2 shows the sulphur contents of crude oil after bioelectrochemical treatment using Desulfovibrio sp. M6: the Table shows that the sulphur content of crude oil was decreased by about 21% by reaction at 30°C for 6 days, but few changes were observed in the reaction mixture without any electrochemical energy supply. $H_2S$ gas was evolved from the cathode compartment, and the amount was increased as a function of reaction time; see Figure 3.

## Table 2

### Sulphur content of crude oil after bioelectrochemical treatment using Desulfovibrio sp. M6

| Treatment | Relative Sulphur Content (%) |
|---|---|
| Control (crude oil) | 100 |
| SRB and methyl viologen | 96 |
| Bioelectrochemical cathode | 79 |

Example 3

An experiment similar to Example 2 was conducted using an electrode of carbon instead of platinum. The surface area of the carbon electrode was $1.10 \text{ cm}^3$. Desulfovibrio sp. M6 was again used as the desulphurisation catalyst. Table 3 shows the sulphur content of crude oil after the bioelectrochemical treatment.

The Table shows that the sulphur content was reduced by about 20.1% after reaction for 6 days; this result was similar to that obtained using a platinum electrode. In the case of reaction without electrochemical energy or methyl viologen, the sulphur content of the crude oil was reduced to a much smaller degree. These results show that electrochemical energy and methyl viologen are essential for the efficient desulphurisation of petroleum.

## Table 3

### Sulphur content of crude oil after bioelectrochemical treatment using a carbon electrode

| Treatment | Relative Sulphur Content (%) |
|---|---|
| Control (crude oil) | 100 |
| SRB and methyl viologen | 96.5 |
| Bioelectrochemical cathode without methyl viologen | 97.8 |
| Electrochemical cathode | 79.9 |

### Example 4

Experiments similar to those of Examples 1 and 2 were performed using diesel oil. The reducing equivalent supplied to the cathode compartment was electrochemical energyor hydrogen gas. Desulfovibrio sp. M6, and 2 mM methyl viologen as an electron mediator, were added, and the reaction was vigorously stirred at 30°C. The results are shown in Table 4.

Table 4 shows the sulphur content of reaction mixtures with electrochemical electron or hydrogen gas was decreased by 23.2% and 39.1%, respectively. These results show that the bioelectrochemical desulphurisation of high sulphur diesel oil was more efficient than that of crude oil.

## Table 4

### Sulphur content of diesel oil after bioelectrochemical treatment using Desulfovibrio sp. M6

| Treatment | Relative Sulphur Content (%) |
|---|---|
| Control (diesel oil) | 100 |
| Electrochemical cathode | 76.8 |
| Hydrogen gas | 60.9 |

### Claims

1. A method for removing sulphur from a sulphur compound-containing fuel, as hydrogen sulphide, which comprises subjecting the fuel to a bioelectrochemical process which is conducted in the cathode compartment of a partitioned cell, in the presence of a bacterium having the ability to catalyse the reduction of a sulphur compound, to produce hydrogen sulphide.

2. A method according to claim 1, which is conducted in the presence of methyl viologen or another electron transfer agent.

3. A method according to claim 1 or claim 2, wherein the bacterium is mesophilic <u>Desulfovibrio</u> sp.

4. A method according to any preceding claim, which is conducted at 30 to 37°C.

5. A method according to any preceding claim, wherein the cathode is of platinum or carbon.

6. A method according to any preceding claim, wherein the bacterium has the ability to catalyse the reduction of dibenzothiophen, to produce $H_2S$.

7. A method according to any preceding claim, wherein anaerobic sludge from an oil tank is substituted for the bacterium.

## Patentansprüche

1. Verfahren zum Entfernen von Schwefel als Schwefelwasserstoff aus einem Schwefelverbindungen enthaltenden Brennstoff, bei dem man den Brennstoff einem bioelektrochemischen Prozeß unterwirft, der im Kathodenraum einer unterteilten Zelle in Gegenwart eines Bakteriums durchgeführt wird, das die Fähigkeit besitzt, die Reduktion einer Schwefelverbindung unter Bildung von Schwefelwasserstoff zu katalysieren.

2. Verfahren nach Anspruch 1, welches in Gegenwart von Methylviologen oder einem anderen Elektronenübertragungsmittel durchgeführt wird.

3. Verfahren nach Anspruch 1 oder 2, worin das Bakterium mesophiles Desulfovibrio sp. ist.

4. Verfahren nach irgendeinem der vorhergehenden Ansprüche, welches bei 30 bis 37°C durchgeführt wird.

5. Verfahren nach irgendeinem der vorhergehenden Ansprüche, worin die Kathode aus Platin oder Kohlenstoff ist.

6. Verfahren nach irgendeinem der vorhergehenden Ansprüche, worin das Bakterium die Fähigkeit besitzt, die Reduktion von Dibenzothiophen unter Bildung von Schwefelwasserstoff zu katalysieren.

7. Verfahren nach irgendeinem der vorhergehenden Ansprüche, worin anaerober Schlamm aus einem Öltank das Bakterium ersetzt.

## Revendications

1. Procédé pour éliminer le soufre sous forme d'hydrogène sulfuré d'un carburant contenant des composés du soufre, qui comprend le fait de soumettre le carburant à un procédé bioélectrochimique qui est mis en oeuvre dans le compartiment cathodique d'une cellule cloisonnée, en présence d'une bactérie capable de catalyser la réduction d'un composé du soufre, pour produire de l'hydrogène sulfuré.

2. Procédé selon la revendication 1, qui est mis en oeuvre en présence de méthylviologène ou d'un autre agent de transfert d'électrons.

3. Procédé selon la revendication 1 ou la revendication 2, dans lequel la bactérie appartient à l'espèce <u>Desulfovibrio</u> mésophile.

4. Procédé selon l'une quelconque des revendications précédentes, qui est mis en oeuvre entre 30 et 37°C.

5. Procédé selon l'une quelconque des revendications précédentes, dans lequel la cathode est en platine ou en carbone.

6. Procédé selon l'une quelconque des revendications précédentes, dans lequel la bactérie est capable de catalyser la réduction du dibenzothiophène pour produire $H_2S$.

7. Procédé selon l'une quelconque des revendications précédentes, dans lequel une suspension anaérobie provenant d'un réservoir de pétrole remplace la bactérie.

Fig. 1

Fig. 2

Fig. 3

Fig. 4

Fig. 5